# EUROPÄISCHE PATENTSCHRIFT

(11) **EP 2 079 717 B1**
(45) Veröffentlichungstag und Bekanntmachung des Hinweises auf die Patenterteilung: **03.03.2010**
(21) Anmeldenummer: 07803418.8
(22) Anmeldetag: 12.09.2007
(51) Int. Cl.: C07D 295/02

(54) **VERFAHREN ZUR KONTINUIERLICHEN DESTILLATIVEN AUFTRENNUNG VON GEMISCHEN ENTHALTEND MORPHOLIN (MO), MONOAMINODIGLYKOL (ADG), AMMONIAK UND WASSER**
METHOD FOR THE CONTINUOUS SEPARATION OF MIXTURES COMPRISING MORPHOLINE (MO), MONOAMINODIGLYCOL (ADG), AMMONIA, AND WATER BY MEANS OF DISTILLATION
PROCÉDÉ DE SÉPARATION PAR DISTILLATION EN CONTINU DE MÉLANGES CONTENANT DE LA MORPHOLINE (MO), DU MONOAMINODIGLYCOL (ADG), DE L'AMMONIAC ET DE L'EAU

(30) Priorität: 28.09.2006 EP 06121434
(43) Veröffentlichungstag der Anmeldung: 22.07.2009
(73) Patentinhaber: BASF SE, 67056 Ludwigshafen (DE)
(72) Erfinder: SCHMIDTKE, Helmut, 64625 Bensheim (DE); BUSSMANN, Oliver, 67071 Ludwigshafen (DE); VERSCH, Ralph, 55234 Eppelsheim (DE); RHEUDE, Udo, 67166 Otterstadt (DE); LEYK, Uwe, 67551 Worms (DE); JULIUS, Manfred, 67117 Limburgerhof (DE); RUDLOFF, Martin, 67161 Gönnheim (DE); HENKES, Erhard, 64683 Einhausen (DE)
(86) Internationale Anmeldenummer: PCT/EP2007/059554
(87) Internationale Veröffentlichungsnummer: WO 2008/037587

(56) Entgegenhaltungen:
- EP-A- 0 696 572
- EP-A- 0 963 975
- US-A- 3 154 544
- US-A- 3 155 657

## Beschreibung

Die vorliegende Erfindung betrifft ein Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) der Formel 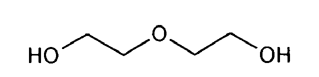 mit Ammoniak.

Aminodiglykol (ADG) [= 2-(2-Aminoethoxy)ethanol = 2,2'-Aminoethoxyethanol, Formel 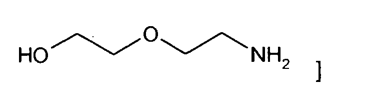 und Morpholin finden u.a. Verwendung als Lösungsmittel, Stabilisatoren, zur Synthese von Chelat-Bildnern, Kunstharzen, Arzneimitteln, Inhibitoren und grenzflächenaktiven Substanzen.
N-Ethyl-morpholin (E-MO) wird u.a. als Katalysator zur Herstellung von Polyurethanschäumen verwendet.

Zur Herstellung von ADG und Morpholin sind in der Literatur zahlreiche Verfahren beschrieben.

Ullmann's Encyclopedia of Industrial Chemistry, 6th Edition, 2000 electronic release, Wiley-VCH Verlag, Rubrik 'cyclic amines' im Kapitel 'aliphatic amines' beschreibt die Synthese von ADG und MO durch Aminierung von DEG unter Wasserstoffdruck und in Gegenwart eines Kobalt- oder Nickelkatalysators (Zitate: EP-A-696 572 (BASF AG), DE-A-1 049 864) oder anderer Katalysatoren (Zitate: DE-A-3 002 342, DE-A-3 125 662 (BASF AG), US 3 155 657).

Die ältere deutsche Patentanmeldung Nr. 102005047458.6 vom 30.09.05 und die ältere europäische (Folge)Patentanmeldung Nr. 06101339.7 vom 06.02.06 (BASF AG) betreffen ein Verfahren zur Herstellung von ADG und Morpholin durch Umsetzung von DEG mit Ammoniak in Gegenwart eines spezifischen Kupfer, Nickel und Kobalt - Heterogenkatalysators sowie allgemein die Aufarbeitung durch mehrstufige Destillation.

Die Patentschrift US 3,155,657 beschreibt ein kontinuierliches verfahren zur Aufarbeitung der Reaktionsgemische von Ammoniak und Diethylenglykol mittels mehrstufiger Destillation.

Zwei parallele europäische Patentanmeldungen mit gleichem Anmeldetag (beide BASF AG) betreffen Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak.

Eine parallele europäische Patentanmeldung mit gleichem Anmeldetag (BASF AG) betrifft ein Verfahren zur Herstellung von ADG in Elektronik-Qualität.

Die Morpholin- und Monoaminodiglykolsynthese ist gekennzeichnet durch die Bildung von einer Vielzahl an Nebenkomponenten. Die Abtrennung der nicht umgesetzten Einsatzstoffe, Wert- sowie der Nebenprodukte erfolgt destillativ, was zu einem erheblichen apparativen und energetischen Aufwand führt.

Der vorliegenden Erfindung lag die Aufgabe zugrunde, unter Überwindung eines Nachteils oder mehrerer Nachteile des Stands der Technik, ein verbessertes wirtschaftliches Verfahren zur Auftrennung von Gemischen enthaltend Morpholin (MO), Monoaminodiglykol (ADG), Ammoniak und Wasser und ggf. N-Ethyl-morpholin (E-MO) und ggf. 1,2-Ethylendiamin (EDA) und ggf. organische Produkte mit einem Siedepunkt > 224,8°C (1,013 bar) aufzufinden. Die einzelnen organischen Komponenten (Amine), insbesondere MO und ADG und ggf. E-MO, sollten dabei in hoher Reinheit und Qualität (z.B. Farbqualität) anfallen.

Demgemäß wurde ein Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak, gefunden, welches dadurch gekennzeichnet ist, dass
in einer ersten Destillationskolonne K10 Ammoniak über Kopf abgetrennt wird,
der Sumpfaustrag von K10 einer zweiten Destillationskolonne K20 zugeführt wird, in der Wasser und organische Produkte über Kopf bei einer Kopftemperatur im Bereich von 45 bis 198°C und einem Druck im Bereich von 0,1 bis 15 bar abgetrennt werden, der Sumpfaustrag von K20 einer dritten Destillationskolonne K30 zugeführt wird, in der MO und organische Produkte mit einem Siedepunkt < 140°C (1,013 bar) über Kopf oder in einem Seitenabzug abgetrennt werden und ADG und organische Produkte mit einem Siedepunkt > 190°C (1,013 bar) über Sumpf abgetrennt werden, und
der MO enthaltende Strom, der bei der Kolonne K30 über Kopf oder im Seitenabzug abgetrennt wird, einer Kolonne K40 zugeführt wird, in der MO über einen Seitenabzug abgetrennt wird, organische Produkte mit einem Siedepunkt ≤ 128°C (1,013 bar), bevorzugt < 128°C (1,013 bar), über Kopf abgetrennt und organische Produkte mit einem Siedepunkt ≥ 128°C (1,013 bar), über Sumpf abgetrennt werden.

Die in Kolonne K40 über Sumpf abgetrennten organischen Produkte werden bevorzugt ganz oder teilweise, besonders bevorzugt ganz, in den Zulauf zur Kolonne K30 zurückgeführt.

Der Sumpf der Kolonne K40 enthält in einer bevorzugten Fahrweise > 94 Gew.-%, besonders > 96 Gew.-%, ganz besonders 98 bis 99 Gew.-%, Morpholin, welches auf den Eingang der Kolonne K30 zurückgeführt wird. Dadurch können eventuell mitgerissene Hochsieder über Sumpf der Kolonne K30 ausgeschleust werden.

Werden die in Kolonne K40 über Sumpf abgetrennten Produkte nicht oder nur teilweise in den Zulauf zur Kolonne K30 zurückgeführt, können sie im Markt für einige Anwendungen auch als MO-Wertprodukt Verwendung finden.

Die Kolonne K10 weist bevorzugt im Bereich von 3 bis 30, besonders im Bereich von 5 bis 20, theoretische Trennstufen auf.
Sie wird bevorzugt bei einem Druck im Bereich von 5 bis 30 bar, besonders 10 bis 20 bar, betrieben.

Bevorzugt befindet sich die Zulaufstelle bei Kolonne K10 im oberen Drittel, bezogen auf die Zahl der theoretischen Trennstufen.

Die Kolonne K20 weist bevorzugt im Bereich von 25 bis 70, besonders im Bereich von 30 bis 60, theoretische Trennstufen auf.
Sie wird bevorzugt bei einem Druck im Bereich von 0,1 bis 10 bar, besonders 0,8 bis 7 bar, betrieben.

Bevorzugt befindet sich die Zulaufstelle bei Kolonne K20 im mittleren Drittel, bezogen auf die Zahl der theoretischen Trennstufen.

In der Kolonne K20 wird bevorzugt Wasser abgetrennt. Mit diesem Wasser werden bevorzugt organische Produkte als Minimumazeotrop über Kopf abgetrennt, welche zum Teil höhere Siedepunkte als das Sumpfprodukt Morpholin haben.

Die Kolonne K30 weist bevorzugt im Bereich von 5 bis 25, besonders im Bereich von 7 bis 20, theoretische Trennstufen auf.
Sie wird bevorzugt bei einem Druck im Bereich von 0,01 bis 5 bar, besonders 0,1 bis 2,5 bar, betrieben.

Bevorzugt befindet sich die Zulaufstelle bei Kolonne K30 im oberen Drittel, bezogen auf die Zahl der theoretischen Trennstufen.
In der alternativen Ausführungsform befindet sich der Seitenabzug bevorzugt 1 bis 8 theoretische Trennstufen, besonders 2 bis 6 theoretische Trennstufen, oberhalb der Zulaufstelle.

Die Kolonne K40 weist bevorzugt im Bereich von 10 bis 80, besonders im Bereich von 15 bis 60, theoretische Trennstufen auf.
Sie wird bevorzugt bei einem Druck im Bereich von 0,01 bis 12 bar, besonders 0,5 bis 6 bar, betrieben.

Bevorzugt befindet sich die Zulaufstelle bei Kolonne K40 im oberen oder mittleren, besonders mittleren, Drittel, bezogen auf die Zahl der theoretischen Trennstufen.
Der gegenüberliegende MO Seitenabzug befindet sich bevorzugt 1 bis 30 theoretische Trennstufen, besonders 2 bis 25 theoretische Trennstufen, unterhalb der Zulaufstelle. In der Kolonne K40 werden organische Produkte mit einem Siedepunkt ≤ 128°C (1,013 bar), bevorzugt < 128°C (1,013 bar), wie z.B. EDA, über Kopf abgetrennt und organische Produkte mit einem Siedepunkt ≥ 128 °C (1,013 bar) über Sumpf abgetrennt.

Die in der Kolonne K40 über Kopf abgetrennten organischen Produkte, insbesondere EDA, können vorteilhaft ganz oder teilweise in den Zulauf zur Kolonne K20 zurückgeführt werden.

In einer weiteren Ausführungsform kann durch weitere destillative Aufreinigung des Kopfdestillats reines EDA als Wertprodukt gewonnen werden.

In einer besonderen Ausführungsform wird der wasser- und organische Produkte enthaltende Strom, der bei der Kolonne K20 über Kopf abgetrennt wird, einer Kolonne K50 zugeführt, in der wässrige N-Ethyl-morpholin-Lösung (wässrige E-MO-Lösung) über Kopf oder einen flüssigen Seitenabzug, wobei sich der flüssige Seitenabzug bevorzugt im oberen Drittel der Kolonne, bezogen auf die Zahl der theoretischen Trennstufen, befindet, und Wasser über Sumpf abgetrennt wird.

Die Kolonne K50 weist bevorzugt im Bereich von 10 bis 50, besonders im Bereich von 15 bis 40, theoretischen Trennstufen auf.
Sie wird bevorzugt bei einem Druck im Bereich von 0,1 bis 16 bar, besonders 0,2 bis 8 bar, betrieben.

Bevorzugt befindet sich die Zulaufstelle bei Kolonne K50 im oberen oder mittleren, besonders im mittleren, Drittel, bezogen auf die Zahl der theoretischen Trennstufen.

Die wässrige N-Ethyl-morpholin-Lösung wird zur Gewinnung des reinen E-MOs zunächst entwässert. Als entwässerndes Mittel wird bevorzugt Natronlauge, z.B. als 40-60 Gew.-%ige wässrige Lösung, besonders 50 Gew.-%ige wässrige Lösung, eingesetzt. Bevorzugt wird die Entwässerung mit der Natronlauge kontinuierlich in einer Extraktionskolonne durchgeführt. Die Extraktionstemperatur liegt bevorzugt zwischen 25-60°C, besonders zwischen 30-55°C. Die Natronlauge wird dabei auf 15-35 Gew.-%, besonders 20-30 Gew.-%, verdünnt.

Nach der Phasentrennung wird die organische Phase in einer kontinuierlichen oder diskontinuierlichen Destillation aufgearbeitet. Die Destillation wird bevorzugt diskontinuierlich in einer Destillationsblase durchgeführt.
Die Kopfprodukte fallen dabei nacheinander an: ggf. Ethylamin, ggf. Ethanol als wässriges Azeotrop, ggf. N-Methyl-morpholin als wässriges Azeotrop, ggf. wasserfreies N-Methyl-morpholin und das Wertprodukt N-Ethyl-morpholin (E-MO).

In einer bevorzugten Ausführungsform wird der Sumpfaustrag von K30 einer Destillationskolonne K60 zugeführt, in der ADG in einem Seitenabzug abgetrennt wird, organische Produkte mit einem Siedepunkt ≤ 224,8°C (1,013 bar) über Kopf abgetrennt und organische Produkte mit einem Siedepunkt > 255°C (1,013 bar) über Sumpf abgetrennt werden.

Die Kolonne K60 weist bevorzugt im Bereich von 20 bis 80, besonders im Bereich von 30 bis 70, theoretische Trennstufen auf.
Sie wird bevorzugt bei einem Druck im Bereich von 0,005 bis 1 bar, besonders 0,01 bis 0,7 bar, betrieben.

Bevorzugt befindet sich die Zulaufstelle bei Kolonne K60 im mittleren oder unteren, besonders mittleren, Drittel, bezogen auf die Zahl der theoretischen Trennstufen. Bevorzugt befindet sich der gegenüberliegende ADG Seitenabzug 1 bis 30, besonders 2 bis 20, theoretische Trennstufen oberhalb der Zulaufstelle.

In einer bevorzugten Ausführungsform werden in der Kolonne K60 über Kopf abgetrennte organische Produkte, wie z.B. N-(2-Aminoethyl)morpholin, 2-(2-Aminoethoxy)-ethylamin, in die Umsetzung von DEG mit Ammoniak zurückgeführt.

Um Aufpegelungen einzelner Komponenten im Kreislauf der Produktionsanlage zu vermeiden, wird bevorzugt ein Teilstrom des im Kolonnenkopf abgetrennten Destillates ausgeschleust. Der Anteil des zurückgeführten Stromes beträgt bevorzugt 40-100 Gew.-%, besonders bevorzugt 50-100 Gew.-%, des im Kolonnenkopf abgetrennten Destillates.

Die in der Kolonne K60 über Sumpf abgetrennten organischen Produkte können vorteilhaft als Mahlhilfsmittel in der Zementindustrie eingesetzt werden.

Der ADG enthaltende Strom, der bei der Kolonne K60 im Seitenabzug abgetrennt wird, wird bevorzugt einer Kolonne K70 zugeführt, in der ADG über einen Seitenabzug abgetrennt wird, organische Produkte mit einem Siedepunkt ≥224,8°C (1,013 bar), besonders > 235°C (1,013 bar), über Sumpf und organische Produkte mit einem Siedepunkt ≤ 224,8°C (1,013 bar) über Kopf abgetrennt werden.

Die Kolonne K70 weist bevorzugt im Bereich von 10 bis 80, besonders im Bereich von 20 bis 70, theoretische Trennstufen auf.
Sie wird bevorzugt bei einem Druck im Bereich von 0,005 bis 1 bar, besonders 0,01 bis 0,7 bar, betrieben.

Bevorzugt befindet sich die Zulaufstelle bei Kolonne K70 im oberen oder mittleren, bevorzugt mittleren, Drittel, bezogen auf die Zahl der theoretischen Trennstufen. Bevorzugt befindet sich der gegenüberliegende ADG Seitenabzug 1 bis 30, besonders 2 bis 25, theoretische Trennstufen oberhalb der Zulaufstelle.

Bevorzugt werden in Kolonne K70 über Sumpf abgetrennte Produkte, wie z.B. DEG, Morpholylaminodiglykol, Morpholinodiglykol, in die Umsetzung von DEG mit Ammoniak zurückgeführt.
(Morpholylaminodiglykol = 4-(2-(2-Aminoethoxy)-ethyl)-morpholin, C₈H₁₈N₂O₂;
Morpholinodiglykol (Morpholinylethoxyethanol) CAS-Nr. 3603-45-0, C₈H₁₇NO₃)

Bevorzugt werden in Kolonne K70 über Kopf abgetrennte Produkte, wie z.B. ADG, N-(2-Aminoethyl)morpholin, 2-(2-Aminoethoxy)ethylamin, in die Umsetzung von DEG mit Ammoniak zurückgeführt.

Der Anteil des zurückgeführten Stromes beträgt bevorzugt 80-100 Gew.-%, besonders bevorzugt 95-100 Gew.-%, des im Kolonnenkopf abgetrennten Destillates.

In einer weiteren besonders bevorzugten Ausführungsform handelt es sich bei der Kolonne K60 um eine Trennwandkolonne (TK).

Die Trennwandkolonne (TK) weist bevorzugt eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (3) und Abtriebsteil (5) auf, wobei die Zuführung des Sumpfaustrags von K30 im oberen oder mittleren Drittel, besonders oberen Drittel, des Zulaufteils (2, 4), bezogen auf die Zahl der theoretischen Trennstufen des Zulaufteils, die Abführung von organischen Produkten mit einem Siedepunkt > 255°C (1,013 bar) über Sumpf, die Abführung von organischen Produkten mit einem Siedepunkt ≤ 224,8°C (1,013 bar) über Kopf, die Abführung von ADG aus dem Kolonnenbereich 1 und optional, in einer besonderen Ausführungsform bevorzugt, die Abführung von dampfförmigen organischen Produkten mit einem Siedepunkt ≥ 224,8°C (1,013 bar), besonders > 235°C (1,013 bar), wie z.B. DEG, aus dem oberen oder mittleren Drittel, besonders oberen Drittel, des Entnahmeteils (3, 5) (Seitenabzug), bezogen auf die Zahl der theoretischen Trennstufen des Entnahmeteils, erfolgt.

In der Kolonne K60 über den dampfförmigen Seitenabzug abgetrennte organische Produkte, wie z.B. DEG, werden bevorzugt in die Umsetzung von DEG mit Ammoniak zurückgeführt.

In einer weiteren vorteilhaften Ausführungsform weist die Trennwandkolonne (TK) eine Trennwand (T) in Kolonnenlängsrichtung unter Ausbildung eines oberen gemeinsamen Kolonnenbereichs (1) und (2), eines Zulaufteils (3, 4) mit Verstärkungsteil (3) und Abtriebsteil (4), sowie eines Teils (5) auf, wobei die Trennwand T bis zum Boden der Kolonne ausgebildet ist, wobei die Zuführung des Sumpfaustrags von K30 im oberen oder mittleren Drittel, besonders oberen Drittel, des Zulaufteils (3, 4), bezogen auf die Zahl der theoretischen Trennstufen des Zulaufteils, die Abführung von DEG und organischen Produkten mit einem Siedepunkt ≥ 224,8°C (1,013 bar), bevorzugt > 235°C (1,013 bar), über Sumpf unterhalb des Teils 5, die Abführung von organischen Produkten mit einem Siedepunkt > 255°C (1,013 bar) (Hochsieder = HS) über Sumpf unterhalb der Teile 3 und 4, die Abführung von organischen Produkten mit einem Siedepunkt ≤ 224,8°C (1,013 bar) über Kopf und die Abführung von ADG aus dem mittleren Teil des oberen gemeinsamen Kolonnenbereichs (1) und (2) (Seitenabzug) erfolgt.

Die Trennwandkolonne K60 weist bevorzugt im Bereich von 30 bis 100, besonders im Bereich von 40 bis 90, theoretische Trennstufen auf.
Sie wird bevorzugt bei einem Druck im Bereich von 0,005 bis 1 bar, besonders 0,01 bis 0,7 bar, betrieben.

Der durch die Trennwand (T) unterteilte Teilbereich der Kolonne TK bestehend aus den Teilbereichen 3, 4 und 5, bzw. 2, 3, 4, und 5, oder jeweils Teilen davon ist bevorzugt mit geordneten Packungen, Füllkörpern und/oder Böden bestückt. Die Trennwand in diesen Teilbereichen ist bevorzugt wärmeisolierend ausgeführt.

In einer bevorzugten Ausführungsform werden in der Kolonne K60 über Kopf abgetrennte organische Produkte, wie z.B. N-(2-Aminoethyl)morpholin, 2-(2-Aminoethoxy)-ethylamin, nicht ausgeschleust, sondern in die Umsetzung von DEG mit Ammoniak zurückgeführt.

In Kolonne K60, mit bis zum Boden der Kolonne ausgebilder Tennwand T, über Sumpf abgetrennte Produkte unterhalb des Teils 5 abgetrennte organische Produkte, wie z.B. DEG, werden bevorzugt in die Umsetzung von DEG mit Ammoniak zurückgeführt.

Der Anteil des zurückgeführten Stromes beträgt bevorzugt 80-100 Gew.-%, besonders bevorzugt 95-100 Gew.-%, des im Kolonnenkopf abgetrennten Destillates.

Das erfindungsgemäße Verfahren ist in besonderen Ausführungsformen bei Einsatz einer Trennwandkolonne (TK) vorteilhaft durch einen geringen Wärmebedarf gegenüber der 2- bzw. 3-Kolonnenverschaltung (K60 - K70 bzw. K80) sowie durch die Reduzierung der Kolonnenanzahl.

In einer besonderen Ausgestaltung des erfindungsgemäßen Verfahrens wird/werden der oder die ADG enthaltende/n Strom/Ströme, der/die bei den Kolonnen K60 und/oder K70 über Kopf abgetrennt wird/werden, ganz oder teilweise einer Kolonne K80 zugeführt, in der ADG und organische Produkte mit einem Siedepunkt ≥ 224,8°C
(1,013 bar) über Sumpf und organische Produkte mit einem Siedepunkt ≤ 224,8°C (1,013 bar) über Kopf abgetrennt werden.

Das im Sumpf anfallende ADG kann als Wertprodukt verwertet werden.

Bevorzugt wird in der Kolonne K80 ADG in besonders reiner Form zusätzlich über einen Seitenabzug abgetrennt.
In diesem Fall werden in Kolonne K80 über Sumpf abgetrennte Produkte bevorzugt in den Zulauf der Kolonnen K60 und/oder K70 zurückgeführt.

In Kolonne K80 über Kopf abgetrennte Produkte werden bevorzugt in die Umsetzung von DEG mit Ammoniak zurückgeführt.

Um Aufpegelungen einzelner Komponenten im Kreislauf der Produktionsanlage zu vermeiden, wird bevorzugt ein Teilstrom des im Kolonnenkopf abgetrennten Destillates ausgeschleust. Der Anteil des zurückgeführten Stromes beträgt bevorzugt 0-100 Gew. %, besonders bevorzugt 0-50 Gew.-%, des im Kolonnenkopf abgetrennten Destillates.

Die Kolonne K80 weist bevorzugt im Bereich von 10 bis 80, besonders im Bereich von 15 bis 60, theoretische Trennstufen auf.
Sie wird bevorzugt bei einem Druck im Bereich von 0,005 bis 3 bar, besonders 0,01 bis 2 bar, betrieben.

Bevorzugt befindet sich die Zulaufstelle bei Kolonne K80 im oberen oder mittleren, bevorzugt oberen, Drittel, bezogen auf die Zahl der theoretischen Trennstufen.
Bevorzugt befindet sich der gegenüberliegende ADG Seitenabzug 1 bis 30, besonders 2 bis 25, theoretische Trennstufen unterhalb der Zulaufstelle.

Das erfindungsgemäße Verfahren ist in besonderen Ausführungsformen durch folgende Wärmeintegrationsmaßnahmen zusätzlich vorteilhaft:
Die Wärme aus den Brüden der K80 kann in der K50 integriert werden.
Die Wärme aus den Brüden der K70 kann in der K50 und/oder K80 integriert werden, bevorzugt in der K 50.
Die Wärme aus den Brüden der K60 kann in der K50 integriert werden.
Die Wärme aus den Brüden der K40 kann in der K20, K50 und/oder K80 integriert werden.
Die Wärme aus den Brüden der Trennwandkolonne K60 kann in der K50 integriert werden.

Diese Wärmeintegration kann wie folgt ausgeführt werden:
Um die anfallende Wärme der Brüden maximal nutzen zu können, wird bevorzugt auf ein Wärmeträgermedium verzichtet und die Brüdenströme werden bevorzugt direkt in den entsprechenden Verdampfern, anstatt des Heizdampfes, kondensiert. Als Verdampfer können jeweils Naturumlaufverdampfer, Zwangsumlaufverdampfer, Zwangsumlaufentspannungsverdampfer, Fallfilmverdampfer, Robertverdampfer, Kettle-Type-Verdampfer, Dünnschichtverdampfer oder Kletterfilmverdampfer eingesetzt werden. Bevorzugt werden jeweils Naturumlaufverdampfer, Zwangsumlaufverdampfer, Zwangsumlaufentspannungsverdampfer, Fallfilmverdampfer, Robertverdampfer oder Kettle-Type-Verdampfer eingesetzt.
Die Restbrüden werden bevorzugt jeweils in einem Nachkondensator verflüssigt.

Weiterhin ist es vorteilhaft, die Reaktionswärme aus der Synthese des aufzutrennenden Gemisches abzuführen, insbesondere über Siedekühlung (Wasserdampf), und in der Destillation zu integrieren. Bei der Synthese sind insbesondere die Ausführungsformen (A) und (B) bevorzugt.

Die Reaktionswärme kann hierbei in den Kolonnen K20, K50, K30, K40, K70 und/oder K80, bevorzugt in den Kolonnen K20, K40 und/oder K80 integriert werden.

Das im erfindungsgemäßen Verfahren eingesetzte Gemisch, enthaltend Morpholin (MO), Monoaminodiglykol (ADG), Ammoniak und Wasser, und ggf. E-MO und ggf. EDA und ggf. organische Produkte mit einem Siedepunkt > 224,8°C (1,013 bar) wurde in einer Ausführungsform (A) bevorzugt erhalten durch Umsetzung von Diethylenglykol (DEG) mit Ammoniak in Gegenwart eines Katalysators, enthaltend Cu, Ni und Co auf Zirkoniumdioxid als Träger.
Katalysatoren dieses Typs sind in EP-A-963 975, EP-A-1 106 600 und WO-A-03/076386 (alle BASF AG) beschrieben.

In einem besonders bevorzugten Katalysator enthält die katalytisch aktive Masse des Katalysators vor der Behandlung mit Wasserstoff im Bereich von 20 bis 65 Gew.-% Zirkoniumdioxid (ZrO₂), 1 bis 30 Gew.-%, sauerstoffhaltige Verbindungen des Kupfers, berechnet als CuO, 15 bis 50 Gew.-%, sauerstoffhaltige Verbindungen des Nickels, berechnet als NiO, und 15 bis 50 Gew.-% sauerstoffhaltige Verbindungen des Kobalts, berechnet als CoO.

Die für die Umsetzung von Diethylenglykol (DEG) mit Ammoniak bevorzugte Temperatur im Reaktor liegt im Bereich von 170 bis 220°C. Bevorzugt ist eine isotherme Reaktorfahrweise. Der für die Umsetzung von Diethylenglykol (DEG) mit Ammoniak bevorzugte Druck liegt im Bereich von 100 bis 200 bar.

Bevorzugt wird die Umsetzung von Diethylenglykol (DEG) mit Ammoniak in Gegenwart von Wasserstoff durchgeführt. Der Wasserstoff wird bevorzugt über einen Hochdruckabscheider als Kreisgas in den Reaktor zurückgeführt.
Das Molverhältnis Ammoniak : DEG liegt bevorzugt im Bereich von 4 bis 6.
Der DEG-Umsatz liegt bevorzugt im Bereich von 55 bis 90 %.

Besonders bevorzugt erfolgt die Herstellung des im erfindungsgemäßen Verfahren eingesetzten Gemisches gemäß der älteren deutsche Patentanmeldung Nr. 102005047458.6 vom 30.09.05 und der älteren europäischen (Folge)Patentanmeldung Nr. 06101339.7 vom 06.02.06 (BASF AG), wonach die Umsetzung von DEG mit Ammoniak in Gegenwart eines spezifischen Katalysatorformkörpers erfolgt und die katalytisch aktive Masse des Katalysators vor der Behandlung mit Wasserstoff sauerstoffhaltige Verbindungen des Aluminiums und/oder Zirkoniums, Kupfers, Nickels und Kobalts enthält.

In einer anderen Ausführungsform (B) wurde das im erfindungsgemäßen Verfahren eingesetzte Gemisch, enthaltend Morpholin (MO), Monoaminodiglykol (ADG), Ammoniak und Wasser, und ggf. E-MO und ggf. EDA und ggf. organische Produkte mit einem Siedepunkt > 224,8°C (1,013 bar), bevorzugt erhalten durch Umsetzung von Diethylenglykol (DEG) mit Ammoniak in Gegenwart eines Katalysators, enthaltend Cu und Ni auf Aluminiumoxid als Träger, wie insbesondere in EP-A-70 397 (BASF AG) beschrieben.
Katalysatoren dieses Typs sind auch in EP-A-514 692 und EP-A-167 872 (beide BASF AG) beschrieben.

In einem hierbei besonders bevorzugten Katalysator enthält die katalytisch aktive Masse des Katalysators vor der Behandlung mit Wasserstoff im Bereich von 25 bis 65 Gew.-% Aluminiumoxid (Al₂O₃), 30 bis 60 Gew.-%, sauerstoffhaltige Verbindungen des Kupfers, berechnet als CuO, und 5 bis 15 Gew.-%, sauerstoffhaltige Verbindungen des Nickels, berechnet als NiO.

Die für die Umsetzung von Diethylenglykol (DEG) mit Ammoniak bevorzugte Temperatur im Reaktor liegt hierbei im Bereich von 190-235°C. Bevorzugt ist eine isotherme Reaktorfahrweise. Der für die Umsetzung von Diethylenglykol (DEG) mit Ammoniak bevorzugte Druck liegt im Bereich von 20 bis 30 bar.

Bevorzugt wird die Umsetzung von Diethylenglykol (DEG) mit Ammoniak in der Gasphase in Gegenwart von Wasserstoff durchgeführt. Der gasförmige Reaktoraustrag wird bevorzugt in einen Wärmeüberträger geführt, in dem bevorzugt eine partielle Kondensation durchgeführt wird (Kondensation von Roh-Morpholin). Die Gasphase, bestehend aus H₂ und NH₃, wird bevorzugt wieder in den DEG-Verdampfer gefahren und danach in den Reaktor.

Das erfindungsgemäße Verfahren ist insbesondere vorteilhaft zur Herstellung von Morpholin (MO)
mit einer Reinheit von ≥ 99,5 Gew.-%, besonders ≥99,6 Gew.-%, z.B. 99,65 bis 99,95 Gew.-%,
einem Gehalt an N-Ethyl-morpholin (E-MO) von ≤ 0,20 Gew.-%, besonders
≤ 0,10 Gew.-%, z.B. 0,01 bis 0,08 Gew.-%,
einem Gehalt an 1,2-Ethylendiamin (EDA) von ≤ 0,30 Gew.-%, besonders
≤ 0,20 Gew.-%, z.B. 0,05 bis 0,15 Gew.-%,
einem Gehalt an 2-Methoxy-ethanol von < 0,50 Gew.-%, besonders < 0,30 Gew.-%, z.B. 0,05 bis 0,25 Gew.-%,
und einem Gehalt an Wasser von ≤ 0,05 Gew.-%, besonders ≤ 0,04 Gew.-%, z.B. 0,01 bis 0,03 Gew.-%.

Ganz besonders ist es vorteilhaft zur Herstellung von Morpholin (MO) mit einer APHA-Farbzahl von ≤ 10, besonders ≤ 8, z.B. 2 bis 7,
und einem Chloridgehalt von ≤ 15 mg/Liter, besonders ≤ 5 mg/Liter, ganz besonders
≤ 1 mg/Liter, z.B. 0,1 bis 0,9 mg/Liter.

Das erfindungsgemäße Verfahren ist weiter insbesondere vorteilhaft zur Herstellung von Monoaminodiglykol (ADG) mit einer Reinheit von ≤ 98,00 Gew.-%, besonders
≥ 98,30 Gew.-%, z.B. 98,50 bis 99,50 Gew.-%,
einem Gehalt an DEG von ≤ 0,40 Gew.-%, besonders ≤ 0,10 Gew.-%, z.B. 0,01 bis 0,08 Gew.-%,
einem Gehalt an Wasser von ≤ 0,20 Gew.-%, besonders ≤ 0,10 Gew.-%, z.B. 0,01 bis 0,08 Gew.-%,
und einer APHA-Farbzahl von ≤ 20, besonders ≤ 15, ganz besonders ≤ 10, z.B. 2 bis 8.

Das erfindungsgemäße Verfahren ist weiter insbesondere vorteilhaft zur Herstellung von N-Ethyl-morpholin (E-MO) mit einer Reinheit von ≥ 98,50 Gew.-%, besonders
≥ 99,00 Gew.-%, z.B. 99,50 bis 99,90 Gew.-%,
einem Gehalt an Wasser von ≤ 0,30 Gew.-%, besonders ≤ 0,20 Gew.-%, z.B. 0,05 bis 0,15 Gew.-%,
und einer APHA-Farbzahl von ≤ 50, besonders ≤ 20, ganz besonders ≤ 10, z.B. 2 bis 8.

APHA-Farbzahlen werden bestimmt gemäß DIN EN 1557.

Der Wassergehalt wird bestimmt gemäß DIN 51777 (K. Fischer).
Der Chloridgehalt wird mittels Ionenchromatographie bestimmt (Detektion von Leitfähigkeit mit chemischer Suppression), nach folgender Methode:
Probenvorbereitung: Ca. 2 g Probe werden in einen Messkolben (10 ml) eingewogen und mit Eluent bis zur Marke aufgefüllt.
Messbedingungen:
   lonenchromatographie-System: Metrohm Modulares System (733)
Vorsäule: z.B. DIONEX AG 12; Trennsäule: z.B. DIONEX AS 12
Eluent: z.B. 2,7 mmol Na₂CO₃, 0,28 mmol/L NaHCO₃ in Wasser
Fluss: 1 ml/min; Dosiervolumen: 100 µl
Detektion: Leitfähigkeit nach chemischer Suppression
Suppressor: Metrohm Modul 753
Regenerent: 50 mmol H₂SO₄ in Reinstwasser, (Fluss ca. 0,4 ml/min)
Kalibration: Extern, überprüft durch Aufstock-Versuche
Bestimmungsgrenze: 0,1 mg/kg Chlorid in der Probe.

Im Wertprodukt Morpholin wird der Gehalt an Morpholin, 1,2-Ethylendiamin, N-Ethylmorpholin, 2-Methoxy-ethanol mittels GC bestimmt (GC-Bedingungen: 30 m DB-1; Temperaturprogramm mit 60°C Anfangstemperatur, 4°C/min. Heizrate, 190°C Endtemperatur).

Im Wertprodukt ADG wird der Gehalt an ADG und DEG in mittels GC bestimmt (GC-Bedingungen: 30 m DB1, Temperaturprogramm mit 100°C Anfangstemperatur, 8°C/min Heizrate, 250 °C Endtemperatur).

Zu den Abbildungen:

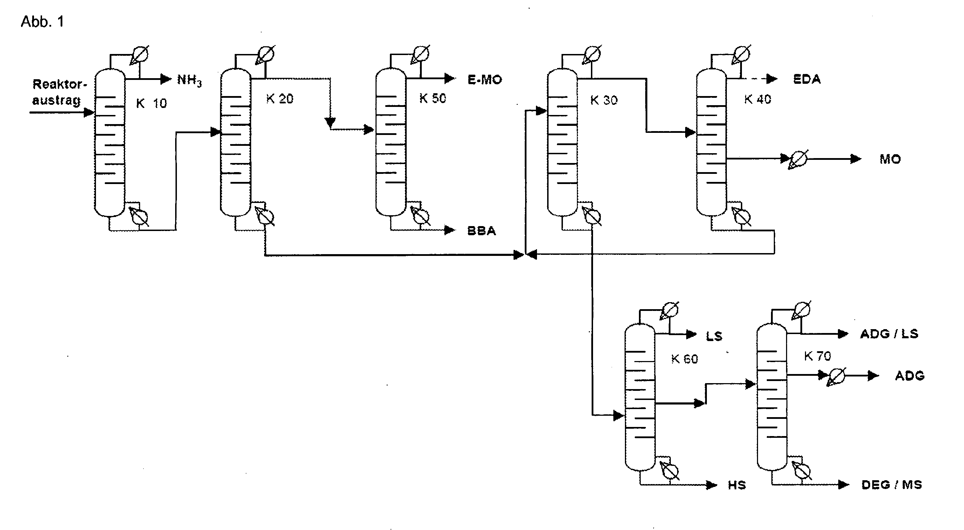

Abbildung 1 zeigt u.a. die erfindungsgemäße Gewinnung von MO und ADG durch eine 7-Kolonnen-Verschaltung.

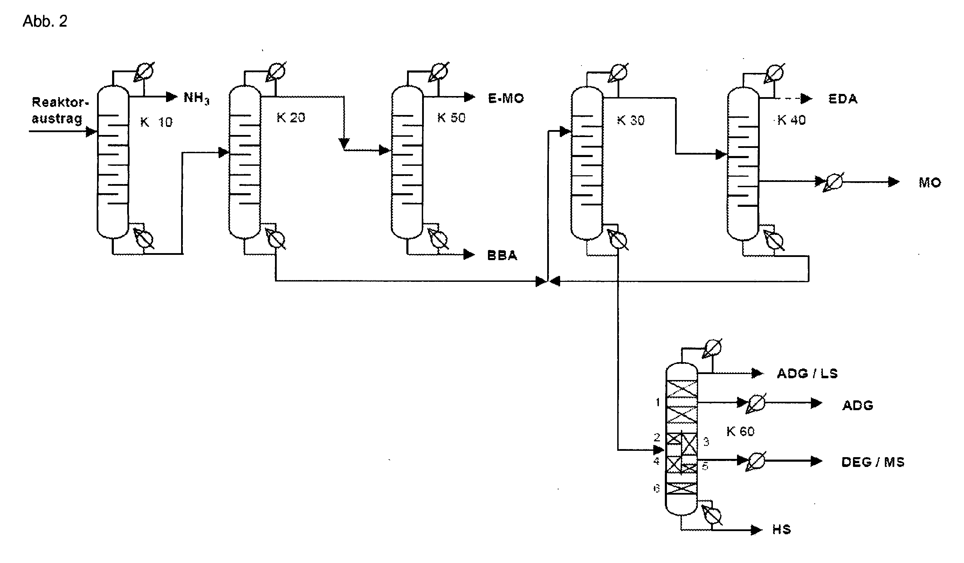

Abbildung 2 zeigt u.a. den Ersatz der Kolonnen K60 - K 70 der 7-Kolonnen-Verschaltung durch eine Trennwandkolonne (TK).

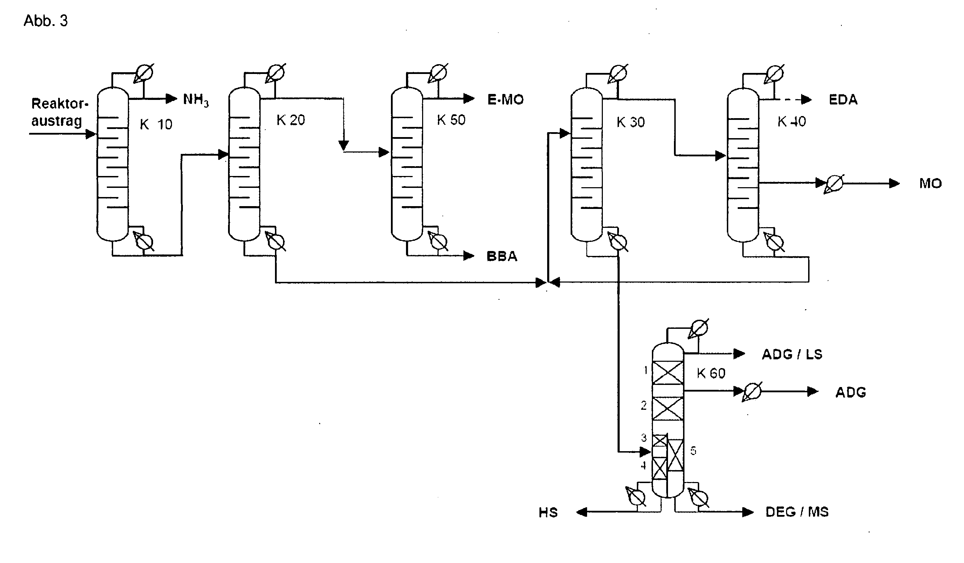

Abbildung 3 zeigt u.a. eine besondere Ausführungsform der Trennwandkolonne K60, in der die Trennwand (T) bis zum Boden der Kolonne ausgebildet ist.

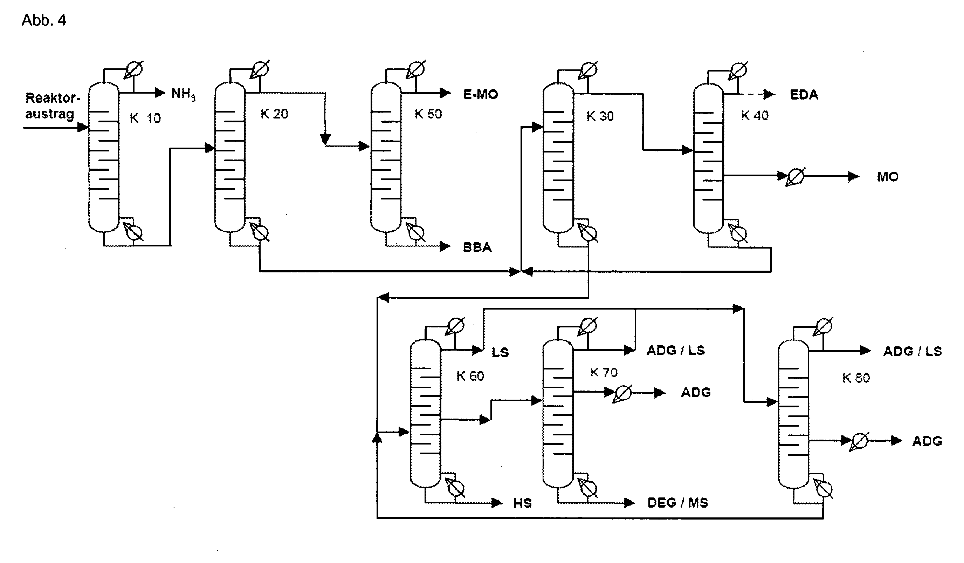

Abbildung 4 zeigt u.a. die erfindungsgemäße Gewinnung von MO und ADG durch eine 8-Kolonnen-Verschaltung.
Abbildung 5 zeigt u.a. die 7-Kolonnen-Verschaltung gemäß des Beispiels, inkl. Rückführströmen und Syntheseteil und Wärmeintegration (B1 = Hochdruckabscheider, B2 = Mitteldruckabscheider, C1 = Reaktor, V1 = Verdichter, W1,2,3 und 4 = Wärmetauscher).
HS = Hochsieder, LS = Leichtsieder, MS = Mittelsieder, BBA = behandlungsbedürftiges Abwasser.

### Beispiele

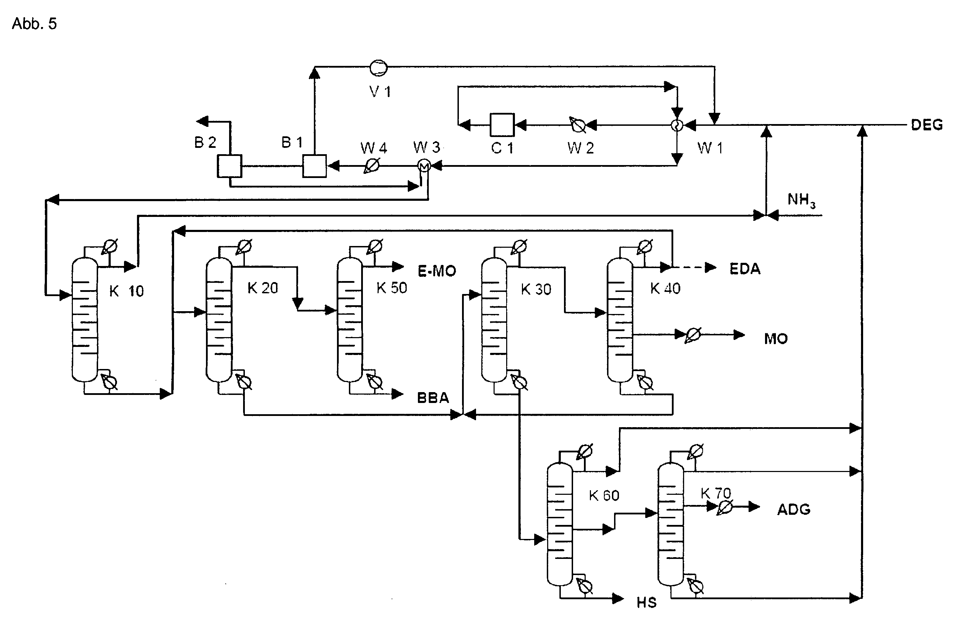

### Beispiel 1 (siehe Abbildung 5)

Diglykol (DEG) wird mit dem Sumpfprodukt der Kolonne K70 (Hauptkomponenten Diglykol und Morpholyl-ADG) und den Kopfprodukten aus den Kolonnen K60 und K70 (Hauptkomponenten: Aminodiglykol, (2-Aminoethyl)morpholin und 2-(2-Aminoethoxy)ethylamin) gemischt und kontinuierlich dem Wärmetauscher W 1 zugeführt. Flüssiges Ammoniak wird mit zurückgeführtem Ammoniak aus der Kolonne K10 gemischt und kontinuierlich dem Wärmetauscher W 1 zugeführt
Beide Ströme werden vor dem Wärmetauscher W 1 mit dem überwiegend aus Wasserstoff bestehendem Kreisgas vermischt. Das Kreisgas wird von dem am Syntheseausgang gelegenen Hochdruckabscheider B 1 mit dem Verdichter V 1 herangeführt. Vom Wärmetauscher W 1 wird das Gemisch mit einer Temperatur von 140°C durch einen Aufheizer W 2 auf 175°C erwärmt und zum Reaktor C 1 gefördert. Am dortigen Festbett-Katalysator erfolgt die Umsetzung des Diglykols zu Aminodiglykol und Morpholin bei einem Druck von 200 bar und Temperaturen bis zu 215°C. Der Reaktoraustrag wird dann in den Wärmetauschern W 1, W 3 und dem Luftkühler W 4 auf 45°C abgekühlt. In dem Hochdruckabscheider B 1 erfolgt die Trennung in Gas- und Flüssigphase. Die Gasphase wird - wie oben beschrieben - als Kreisgas zum Wärmetauscher W 1 geführt.

Die flüssige Phase wird aus dem Hochdruckabscheider B 1 in den Mitteldruckabscheider B 2 auf 25 bar entspannt. Das dort frei werdende sogenannte Sprudelgas wird zur NH₃-Rückgewinnung in einen Absorber geleitet. Die zu ergänzende Wasserstoffmenge wird dem Netz entnommen und am Synthesezulauf eingespeist.

Aus dem Mitteldruckabscheider B 2 gelangt das Reaktionsgemisch dann über den Wärmetauscher W 3 in die Kolonne K10.

### Ammoniakabtrennung (K10)

In der Kolonne K10 wird das Ammoniak mit einer Reinheit > 99,9 % bei einem Kopfdruck von 16 bar_{abs.} aus dem Zulauf abdestilliert und auf den Reaktoreingang zurückgeführt. Das Abgas ist an einen Absorber angeschlossen. Der ammoniakfreie Sumpf mit einer Temperatur von 220°C wird zur Abtrennung von Wasser in die Kolonne K20 entspannt. Die K10 hat 17 theoretische Trennstufen, der Zulauf erfolgt auf Stufe 14.

### Wasserabtrennung (K20)

In der Kolonne K20 wird das Reaktionswasser bei Normaldruck abgetrennt. Das Destillat, das 98 Gew.-% Wasser und 2 Gew.-% Leichtsieder (überwiegend Ethylmorpholin) enthält, wird zur Kolonne K50 geführt. Darüberhinaus wird der Kolonne K20 das Kopfprodukt der Morpholin-Reindestillation K40 (Hauptkomponenten: 1,2-Ethylendiamin, Morpholin und Wasser) zugeführt. Der weitgehend wasserfreie Sumpf der K 20 mit einer Temperatur von 158°C (Hauptkomponenten: Morpholin, Aminodiglykol, Diglykol und hochsiedender Rückstand) wird zur Kolonne K30 entspannt. Die K20 hat 56 theoretische Trennstufen, der Zulauf erfolgt auf Stufe 25.

### LS/HS-Abtrennung (K30)

In der Kolonne K30 wird der Zulauf, der aus dem Sumpfabzug der Kolonne K20 und dem zurückgeführten Sumpfabzug der Reindestillation K40 besteht, bei einem Kopfdruck von 550 mbar in eine Leichtsiederfraktion (Hauptkomponente: Morpholin) und eine Hochsiederfraktion mit einer Sumpftemperatur von 213°C (Hauptkomponenten: Aminodiglykol, Diglykol und hochsiedender Rückstand) aufgetrennt. Der Sumpf wird der Kolonne K60 zugeführt. Das Kondensat mit 95 Gew.-% Morpholin, 4,5 Gew.-% 1,2-Ethylendiamin sowie 2-Methoxy-ethanol und Wasser wird der Kolonne K40 zugeführt.
Die K30 hat 17 theoretische Trennstufen, der Zulauf erfolgt auf Stufe 13.

### Morpholin-Reindestillation (K40)

In der Kolonne K40 wird bei einem Kopfdruck von 2,2 bar_{abs}. Morpholin in einem gasförmigen Seitenabzug mit einer Konzentration > 99,6 Gew.-% Morpholin und einem Gehalt an 1,2-Ethylendiamin (EDA) von < 0,10 Gew.-% abgetrennt. Das Kopfdestillat (Hauptkomponenten: 1,2-Ethylendiamin, Morpholin und Wasser) wird zur K20 zurückgeführt bzw. nach Anreicherung von Ethylendiamin diskontinuierlich über einen Behälter ausgeschleust. Der Sumpf der Kolonne K40 mit einer Temperatur von 160°C (Morpholin mit schwerer siedenden Nebenkomponenten) geht zurück zur K30.
Die K40 hat 42 theoretische Trennstufen, der Zulauf erfolgt auf Stufe 18, der Seitenabzug auf Stufe 3.

### Ethylmorpholin-Destillation (K50)

In der Kolonne K50 wird bei Normaldruck N-Ethylmorpholin als Azeotrop mit Wasser aus dem Zulauf abgetrennt. Der Sumpf der Kolonne mit einer Temperatur von 103°C wird ausgeschleust. Die K50 hat 21 theoretische Trennstufen, der Zulauf erfolgt auf Stufe 11.

### Rückstandsabtrennung (K60)

In der Kolonne K60 werden aus dem Zulauf Aminodiglykol und Diglykol gemeinsam als flüssiger Seitenabzug bei einem Kopfdruck von 60 mbar abgetrennt und zur Kolonne K70 gefördert. Das Destillat der Kolonne (Hauptkomponenten: Aminoethoxyethylamin, Aminoethylmorpholin, Aminodiglykol) wird auf den Reaktoreingang zurückgefördert. Der Sumpf der Kolonne mit einer Sumpftemperatur von 204°C wird aus dem Verfahren ausgeschleust. Die K60 hat 42 theoretische Trennstufen, der Zulauf erfolgt auf Stufe 20, der Seitenabzug auf Stufe 36.

### Aminodiglykol-Destillation (K70)

In der Kolonne K70 wird aus dem Zulauf Aminodiglykol mit einer Reinheit
> 98,0 Gew.-% und einem Gehalt an Wasser von < 0,10 Gew.-% bei einem Kopfdruck von 80 mbar als flüssiger Seitenabzug abgetrennt. Das Kondensat der Kolonne (Hauptkomponenten: Aminodiglykol, (2-Aminoethyl)morpholin und 2-(2-Aminoethoxy)-ethylamin) wird auf den Reaktoreingang zurückgeführt. Der Sumpf der Kolonne mit einer Sumpftemperatur von 238°C (87 Gew.-% DEG, 11 Gew.-% Morpholyl-ADG und 2 Gew.-% Hochsiedern) wird ebenfalls auf den Reaktoreingang zurückgeführt. Die K70 hat 42 theoretische Trennstufen, der Zulauf erfolgt auf Stufe 25, der Seitenabzug auf Stufe 35.

Zählweise der Stufen in den Kolonnen ist von unten nach oben.

## Patentansprüche

1. Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak, **dadurch gekennzeichnet, dass**
in einer ersten Destillationskolonne K10 Ammoniak über Kopf abgetrennt wird, der Sumpfaustrag von K10 einer zweiten Destillationskolonne K20 zugeführt wird, in der Wasser und organische Produkte über Kopf bei einer Kopftemperatur im Bereich von 45 bis 198°C und einem Druck im Bereich von 0,1 bis 15 bar abgetrennt werden,
der Sumpfaustrag von K20 einer dritten Destillationskolonne K30 zugeführt wird, in der MO und organische Produkte mit einem Siedepunkt < 140°C (1,013 bar) über Kopf oder in einem Seitenabzug abgetrennt werden und ADG und organische Produkte mit einem Siedepunkt > 190°C (1,013 bar) über Sumpf abgetrennt werden, und
der MO enthaltende Strom, der bei der Kolonne K30 über Kopf oder im Seitenabzug abgetrennt wird, einer Kolonne K40 zugeführt wird, in der MO über einen Seitenabzug abgetrennt wird, organische Produkte mit einem Siedepunkt
≤ 128 °C (1,013 bar) über Kopf abgetrennt und organische Produkte mit einem Siedepunkt ≥ 128 °C (1,013 bar) über Sumpf abgetrennt werden.

2. Verfahren nach dem vorhergehenden Anspruch, **dadurch gekennzeichnet, dass** die in Kolonne K40 über Sumpf abgetrennten Produkte ganz oder teilweise in den Zulauf zur Kolonne K30 zurückgeführt werden.

3. Verfahren nach einem der beiden vorhergehenden Ansprüche, **dadurch gekennzeichnet, dass** die in Kolonne K40 über Kopf abgetrennten Produkte ganz oder teilweise in den Zulauf zur Kolonne K20 zurückgeführt werden.

4. Verfahren nach einem der vorhergehenden Ansprüche, **dadurch gekennzeichnet, dass** die Kolonne K10 im Bereich von 3 bis 30 theoretische Trennstufen aufweist und bei einem Druck im Bereich von 5 bis 30 bar betrieben wird.

5. Verfahren nach einem der vorhergehenden Ansprüche, **dadurch gekennzeichnet, dass** die Kolonne K20 im Bereich von 25 bis 70 theoretische Trennstufen aufweist und bei einem Druck im Bereich von 0,1 bis 10 bar betrieben wird.

6. Verfahren nach einem der vorhergehenden Ansprüche, **dadurch gekennzeichnet, dass** die Kolonne K30 im Bereich von 5 bis 25 theoretische Trennstufen aufweist und bei einem Druck im Bereich von 0,01 bis 5 bar betrieben wird.

7. Verfahren nach einem der vorhergehenden Ansprüche, **dadurch gekennzeichnet, dass** die Kolonne K40 im Bereich von 10 bis 80 theoretischen Trennstufen aufweist und bei einem Druck im Bereich von 0,01 bis 12 bar betrieben wird.

8. Verfahren nach einem der vorhergehenden Ansprüche, **dadurch gekennzeichnet, dass** sich die Zulaufstelle bei Kolonne K10 im oberen Drittel befindet, bezogen auf die Zahl der theoretischen Trennstufen.

9. Verfahren nach einem der vorhergehenden Ansprüche, **dadurch gekennzeichnet, dass** sich die Zulaufstelle bei Kolonne K20 im mittleren Drittel befindet, bezogen auf die Zahl der theoretischen Trennstufen.

10. Verfahren nach einem der vorhergehenden Ansprüche, **dadurch gekennzeichnet, dass** sich die Zulaufstelle bei Kolonne K30 im oberen Drittel befindet, bezogen auf die Zahl der theoretischen Trennstufen.

11. Verfahren nach einem der vorhergehenden Ansprüche, **dadurch gekennzeichnet, dass** sich die Zulaufstelle bei Kolonne K40 im oberen oder mittleren Drittel befindet, bezogen auf die Zahl der theoretischen Trennstufen, und sich der gegenüberliegende MO Seitenabzug 1 bis 30 theoretische Trennstufen unterhalb der Zulaufstelle befindet.

12. Verfahren nach einem der vorhergehenden Ansprüche, **dadurch gekennzeichnet, dass** der wasser- und organische Produkte enthaltende Strom, der bei der Kolonne K20 über Kopf abgetrennt wird, einer Kolonne K50 zugeführt wird, in der wässrige N-Ethyl-morpholin-Lösung (wässrige E-MO-Lösung) über Kopf oder einen flüssigen Seitenabzug und Wasser über Sumpf abgetrennt wird.

13. Verfahren nach dem vorhergehenden Anspruch, **dadurch gekennzeichnet, dass** die Kolonne K50 im Bereich von 10 bis 50 theoretischen Trennstufen aufweist und bei einem Druck im Bereich von 0,1 bis 16 bar betrieben wird.

14. Verfahren nach einem der beiden vorhergehenden Ansprüche, **dadurch gekennzeichnet, dass** sich die Zulaufstelle bei Kolonne K50 im oberen oder mittleren Drittel befindet, bezogen auf die Zahl der theoretischen Trennstufen.

15. Verfahren nach einem der drei vorhergehenden Ansprüche, **dadurch gekennzeichnet, dass** die wässrige N-Ethyl-morpholin-Lösung entwässert und danach die hierbei entstehende organische Phase durch Destillation zum Wertprodukt aufkonzentriert wird.

16. Verfahren nach einem der vorhergehenden Ansprüche, **dadurch gekennzeichnet, dass** der Sumpfaustrag von K30 einer Destillationskolonne K60 zugeführt wird, in der ADG in einem Seitenabzug abgetrennt wird, organische Produkte mit einem Siedepunkt ≤ 224,8°C (1,013 bar) über Kopf abgetrennt und organische Produkte mit einem Siedepunkt > 255 °C (1,013 bar) über Sumpf abgetrennt werden.

17. Verfahren nach dem vorhergehenden Anspruch, **dadurch gekennzeichnet, dass** die Kolonne K60 im Bereich von 20 bis 80 theoretischen Trennstufen aufweist und bei einem Druck im Bereich von 0,005 bis 1 bar betrieben wird.

18. Verfahren nach einem der beiden vorhergehenden Ansprüche, **dadurch gekennzeichnet, dass** sich die Zulaufstelle bei Kolonne K60 im mittleren oder unteren Drittel befindet, bezogen auf die Zahl der theoretischen Trennstufen, und sich der gegenüberliegende ADG Seitenabzug 1 bis 30 theoretische Trennstufen oberhalb der Zulaufstelle befindet.

19. Verfahren nach einem der drei vorhergehenden Ansprüche, **dadurch gekennzeichnet, dass** in der Kolonne K60 über Kopf abgetrennte organische Produkte ausgeschleust oder in die Umsetzung von DEG mit Ammoniak zurückgeführt werden.

20. Verfahren nach einem der vier vorhergehenden Ansprüche, **dadurch gekennzeichnet, dass** der ADG enthaltende Strom, der bei der Kolonne K60 im Seitenabzug abgetrennt wird, einer Kolonne K70 zugeführt wird, in der ADG über einen Seitenabzug abgetrennt wird, organische Produkte mit einem Siedepunkt
≥ 224,8°C (1,013 bar) über Sumpf und organische Produkte mit einem Siedepunkt ≤ 224,8°C (1,013 bar) über Kopf abgetrennt werden.

21. Verfahren nach dem vorhergehenden Anspruch, **dadurch gekennzeichnet, dass** die Kolonne K70 im Bereich von 10 bis 80 theoretischen Trennstufen aufweist und bei einem Druck im Bereich von 0,005 bis 1 bar betrieben wird.

22. Verfahren nach einem der beiden vorhergehenden Ansprüche, **dadurch gekennzeichnet, dass** sich die Zulaufstelle bei Kolonne K70 im oberen oder mittleren Drittel befindet, bezogen auf die Zahl der theoretischen Trennstufen, und sich der gegenüberliegende ADG Seitenabzug 1 bis 30 theoretische Trennstufen oberhalb der Zulaufstelle befindet.

23. Verfahren nach einem der drei vorhergehenden Ansprüche, **dadurch gekennzeichnet, dass** in Kolonne K70 über Sumpf abgetrennte Produkte in die Umsetzung von DEG mit Ammoniak zurückgeführt werden.

24. Verfahren nach einem der vier vorhergehenden Ansprüche, **dadurch gekennzeichnet, dass** in Kolonne K70 über Kopf abgetrennte Produkte in die Umsetzung von DEG mit Ammoniak zurückgeführt werden.

25. Verfahren nach Anspruch 16, **dadurch gekennzeichnet, dass** es sich bei der Kolonne K60 um eine Trennwandkolonne (TK) handelt.

26. Verfahren nach dem vorhergehenden Anspruch, **dadurch gekennzeichnet, dass** die Trennwandkolonne (TK) eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (3) und Abtriebsteil (5) aufweist, wobei die Zuführung des Sumpfaustrags von K30 im oberen oder mittleren Drittel des Zulaufteils (2, 4), bezogen auf die Zahl der theoretischen Trennstufen des Zulaufteils, die Abführung von organischen Produkten mit einem Siedepunkt > 255°C (1,013 bar) über Sumpf, die Abführung von organischen Produkten mit einem Siedepunkt ≤ 224,8°C (1,013 bar) über Kopf, die Abführung von ADG aus dem Kolonnenbereich 1 und optional die Abführung von dampfförmigen organischen Produkten mit einem Siedepunkt
≥ 224,8°C (1,013 bar), wie DEG, aus dem oberen oder mittleren Drittel des Entnahmeteils (3, 5) (Seitenabzug), bezogen auf die Zahl der theoretischen Trennstufen des Entnahmeteils, erfolgt.

27. Verfahren nach dem vorhergehenden Anspruch, **dadurch gekennzeichnet, dass** über den dampfförmigen Seitenabzug abgetrennte Produkte in die Umsetzung von DEG mit Ammoniak zurückgeführt werden.

28. Verfahren nach Anspruch 25, **dadurch gekennzeichnet, dass** die Trennwandkolonne (TK) eine Trennwand (T) in Kolonnenlängsrichtung unter Ausbildung eines oberen gemeinsamen Kolonnenbereichs (1) und (2), eines Zulaufteils (3, 4) mit Verstärkungsteil (3) und Abtriebsteil (4), sowie eines Teils (5) aufweist, wobei die Trennwand T bis zum Boden der Kolonne ausgebildet ist, wobei die Zuführung des Sumpfaustrags von K30 im oberen oder mittleren Drittel des Zulaufteils (3, 4), bezogen auf die Zahl der theoretischen Trennstufen des Zulaufteils, die Abführung von DEG und organischen Produkten mit einem Siedepunkt ≥ 224,8°C (1,013 bar) über Sumpf unterhalb des Teils 5, die Abführung von organischen Produkten mit einem Siedepunkt > 255°C (1,013 bar) über Sumpf unterhalb der Teile 3 und 4, die Abführung von organischen Produkten mit einem Siedepunkt ≤ 224,8 °C (1,013 bar) über Kopf und die Abführung von ADG aus dem mittleren Teil des oberen gemeinsamen Kolonnenbereichs (1) und (2) (Seitenabzug) erfolgt.

29. Verfahren nach dem vorhergehenden Anspruch, **dadurch gekennzeichnet, dass** über Sumpf abgetrennte Produkte unterhalb des Teils 5 in die Umsetzung von DEG mit Ammoniak zurückgeführt werden.

30. Verfahren nach einem der fünf vorhergehenden Ansprüche, **dadurch gekennzeichnet, dass** die Trennwandkolonne K60 im Bereich von 30 bis 100 theoretischen Trennstufen aufweist und bei einem Druck im Bereich von 0,005 bis 1 bar betrieben wird.

31. Verfahren nach einem der sechs vorhergehenden Ansprüche, **dadurch gekennzeichnet, dass** der durch die Trennwand (T) unterteilte Teilbereich der Kolonne (TK) bestehend aus den Teilbereichen 3, 4 und 5, bzw. 2, 3, 4, und 5, oder jeweils Teilen davon mit geordneten Packungen, Füllkörpern und/oder Böden bestückt ist und die Trennwand in diesen Teilbereichen wärmeisolierend ausgeführt ist.

32. Verfahren nach einem der sieben vorhergehenden Ansprüche, **dadurch gekennzeichnet, dass** über Kopf abgetrennte organische Produkte in die Umsetzung von DEG mit Ammoniak zurückgeführt werden.

33. Verfahren nach einem der Ansprüche 16 bis 32, **dadurch gekennzeichnet, dass** der oder die ADG enthaltende/n Strom/Ströme, der/die bei den Kolonnen K60 und/oder K70 über Kopf abgetrennt wird/werden, ganz oder teilweise einer Kolonne K80 zugeführt wird/werden, in der ADG und organische Produkte mit einem Siedepunkt ≥ 224,8°C (1,013 bar) über Sumpf und organische Produkte mit einem Siedepunkt ≤ 224,8°C (1,013 bar) über Kopf abgetrennt werden.

34. Verfahren nach dem vorhergehenden Anspruch, **dadurch gekennzeichnet, dass** in der Kolonne K80 ADG zusätzlich über einen Seitenabzug abgetrennt wird.

35. Verfahren nach einem der beiden vorhergehenden Ansprüche, **dadurch gekennzeichnet, dass** in Kolonne K80 über Sumpf abgetrennte Produkte in den Zulauf der Kolonnen K60 und/oder K70 zurückgeführt werden.

36. Verfahren nach einem der drei vorhergehenden Ansprüche, **dadurch gekennzeichnet, dass** in Kolonne K80 über Kopf abgetrennte Produkte ausgeschleust oder in die Umsetzung von DEG mit Ammoniak zurückgeführt werden.

37. Verfahren nach einem der vier vorhergehenden Ansprüche, **dadurch gekennzeichnet, dass** die Kolonne K80 im Bereich von 10 bis 80 theoretischen Trennstufen aufweist und bei einem Druck im Bereich von 0,005 bis 3 bar betrieben wird.

38. Verfahren nach einem der fünf vorhergehenden Ansprüche, **dadurch gekennzeichnet, dass** sich die Zulaufstelle bei Kolonne K80 im oberen oder mittleren Drittel befindet, bezogen auf die Zahl der theoretischen Trennstufen, und sich der gegenüberliegende ADG Seitenabzug 1 bis 30 theoretische Trennstufen unterhalb der Zulaufstelle befindet.

39. Verfahren nach einem der vorhergehenden Ansprüche zur Auftrennung von Gemischen enthaltend Morpholin (MO), Monoaminodiglykol (ADG), Ammoniak, Wasser, N-Ethyl-morpholin (E-MO), 1,2-Ethylendiamin (EDA) und organische Produkte mit einem Siedepunkt > 224,8 °C (1,013 bar).

40. Verfahren nach einem der vorhergehenden Ansprüche, **dadurch gekennzeichnet, dass** das Gemisch, enthaltend Morpholin (MO), Monoaminodiglykol (ADG), Ammoniak und Wasser, erhalten wurde durch Umsetzung von Diethylenglykol (DEG) mit Ammoniak in Gegenwart eines Heterogenkatalysators, enthaltend Cu, Ni und Co auf Zirkoniumdioxid als Träger oder in Gegenwart eines Heterogenkatalysators, enthaltend Cu und Ni auf Aluminiumoxid als Träger.

41. Verfahren nach einem der vorhergehenden Ansprüche zur Herstellung von Morpholin (MO) mit einer Reinheit von ≥ 99,5 Gew.-%, einem Gehalt an N-Ethylmorpholin (E-MO) von ≤ 0,20 Gew.-%, einem Gehalt an 1,2-Ethylendiamin (EDA) von ≤ 0,30 Gew.-%, einem Gehalt an 2-Methoxy-ethanol von < 0,50 Gew.-% und einem Gehalt an Wasser von ≤ 0,05 Gew.-%.

42. Verfahren nach einem der vorhergehenden Ansprüche zur Herstellung von Morpholin (MO) mit einer APHA-Farbzahl von ≤ 10 und einem Chloridgehalt von ≤ 15 mg/Liter.

43. Verfahren nach einem der Ansprüche 20 bis 42 zur Herstellung von Monoaminodiglykol (ADG) mit einer Reinheit von ≥ 98,00 Gew.-%, einem Gehalt an DEG von ≤ 0,40 Gew.-%, einem Gehalt an Wasser von ≤ 0,20 Gew.-% und einer APHA-Farbzahl von ≤ 20.

44. Verfahren nach einem der Ansprüche 15 bis 43 zur Herstellung von N-Ethylmorpholin (E-MO) mit einer Reinheit von ≥ 98,50 Gew.-%, einem Gehalt an Wasser von ≤ 0,30 Gew.-% und einer APHA-Farbzahl von ≤ 50.

## Claims

1. A process for the continuous fractional distillation of mixtures comprising morpholine (MO), monoaminodiglycol (ADG), ammonia and water obtained by reaction of diethylene glycol (DEG) with ammonia, which comprises
separating off ammonia at the top of a first distillation column K10,
feeding the bottoms from K10 to a second distillation column K20 in which water and organic products are separated off at the top at a temperature at the top in the range from 45 to 198°C and a pressure in the range from 0.1 to 15 bar, feeding the bottoms from K20 to a third distillation column K30 in which MO and organic products having a boiling point of < 140°C (1.013 bar) are separated off at the top or at a side offtake and ADG and organic products having a boiling point of > 190°C (1.013 bar) are separated off at the bottom, and
feeding the MO-comprising stream which is separated off at the top or at a side offtake of the column K30 to a column K40 in which MO is separated off at a side offtake, organic products having a boiling point of ≤ 128°C (1.013 bar) are separated off at the top and organic products having a boiling point of ≥ 128°C (1.013 bar) are separated off at the bottom.

2. The process according to the preceding claim, wherein the products separated off at the bottom of column K40 are recirculated in their entirety or in part to the feed to the column K30.

3. The process according to either of the two preceding claims, wherein the products separated off at the top of column K40 are recirculated in their entirety or in part to the feed to the column K20.

4. The process according to any of the preceding claims, wherein the column K10 has from 3 to 30 theoretical plates and is operated at a pressure in the range from 5 to 30 bar.

5. The process according to any of the preceding claims, wherein the column K20 has from 25 to 70 theoretical plates and is operated at a pressure in the range from 0.1 to 10 bar.

6. The process according to any of the preceding claims, wherein the column K30 has from 5 to 25 theoretical plates and is operated at a pressure in the range from 0.01 to 5 bar.

7. The process according to any of the preceding claims, wherein the column K40 has from 10 to 80 theoretical plates and is operated at a pressure in the range from 0.01 to 12 bar.

8. The process according to any of the preceding claims, wherein the feed point of column K10 is located in the upper third, based on the number of theoretical plates.

9. The process according to any of the preceding claims, wherein the feed point of column K20 is located in the middle third, based on the number of theoretical plates.

10. The process according to any of the preceding claims, wherein the feed point of column K30 is located in the upper third, based on the number of theoretical plates.

11. The process according to any of the preceding claims, wherein the feed point of column K40 is located in the upper or middle third, based on the number of theoretical plates, and the MO side offtake located opposite the feed point is located from 1 to 30 theoretical plates below the feed point.

12. The process according to any of the preceding claims, wherein the stream comprising water and organic products which is separated off at the top of the column K20 is fed to a column K50 in which aqueous N-ethylmorpholine solution (aqueous E-MO solution) is separated off at the top or at a side offtake for liquid and water is separated off at the bottom.

13. The process according to the preceding claim, wherein the column K50 has from 10 to 50 theoretical plates and is operated at a pressure in the range from 0.1 to 16 bar.

14. The process according to either of the two preceding claims, wherein the feed point of column K50 is located in the upper or middle third, based on the number of theoretical plates.

15. The process according to any of the three preceding claims, wherein the aqueous N-ethylmorpholine solution is dewatered and the organic phase formed here is then concentrated by distillation to give the desired product.

16. The process according to any of the preceding claims, wherein the bottoms from K30 are fed to a distillation column K60 in which ADG is separated off at a side offtake, organic products having a boiling point of ≤ 224.8°C (1.013 bar) are separated off at the top and organic products having a boiling point of > 255°C (1.013 bar) are separated off at the bottom.

17. The process according to the preceding claim, wherein the column K60 has from 20 to 80 theoretical plates and is operated at a pressure in the range from 0.005 to 1 bar.

18. The process according to either of the two preceding claims, wherein the feed point of column K60 is located in the middle or lower third, based on the number of theoretical plates, and the ADG side offtake located opposite the feed point is located from 1 to 30 theoretical plates above the feed point.

19. The process according to any of the three preceding claims, wherein organic products separated off at the top of the column K60 are discharged or recirculated to the reaction of DEG with ammonia.

20. The process according to any of the four preceding claims, wherein the ADG-comprising stream separated off at the side offtake of the column K60 is fed to a column K70 in which ADG is separated off at a side offtake, organic products having a boiling point of ≥ 224.8°C (1.013 bar) are separated off at the bottom and organic products having a boiling point of ≤ 224.8°C (1.013 bar) are separated off at the top.

21. The process according to the preceding claim, wherein the column K70 has from 10 to 80 theoretical plates and is operated at a pressure in the range from 0.005 to 1 bar.

22. The process according to either of the two preceding claims, wherein the feed point of column K70 is located in the upper or middle third, based on the number of theoretical plates, and the ADG side offtake located opposite the feed point is located from 1 to 30 theoretical plates above the feed point.

23. The process according to any of the three preceding claims, wherein products separated off at the bottom of column K70 are recirculated to the reaction of DEG with ammonia.

24. The process according to any of the four preceding claims, wherein products separated off at the top of column K70 are recirculated to the reaction of DEG with ammonia.

25. The process according to claim 16, wherein the column K60 is a dividing wall column (DWC).

26. The process according to the preceding claim, wherein the dividing wall column (DWC) has a dividing wall (DW) in the longitudinal direction of the column to form an upper combined column region (1), a lower combined column region (6), an inflow part (2, 4) having an enrichment section (2) and a stripping section (4), and also an offtake part (3, 5) having an enrichment section (3) and a stripping section (5), with the bottoms from K30 being fed in in the upper or middle third of the inflow part (2, 4), based on the number of theoretical plates of the inflow part, organic products having a boiling point of > 255°C (1.013 bar) being discharged at the bottom, organic products having a boiling point of ≤ 224.8°C (1.013 bar) being discharged at the top, ADG being discharged from the column region 1 and optionally gaseous organic products having a boiling point of ≥ 224.8°C (1.013 bar), e.g. DEG, being discharged from the upper or middle third of the offtake part (3, 5) (side offtake), based on the number of theoretical plates of the offtake part.

27. The process according to the preceding claim, wherein products separated off in gaseous form at the side offtake are recirculated to the reaction of DEG with ammonia.

28. The process according to claim 25, wherein the dividing wall column (DWC) has a dividing wall (DW) in the longitudinal direction of the column to form an upper combined column region (1) and (2), an inflow part (3, 4) having an enrichment section (3) and a stripping section (4), and also a part (5), with the dividing wall DW extending to the bottom of the column and the bottoms from K30 being fed in in the upper or middle third of the inflow part (3, 4), based on the number of theoretical plates of the inflow part, DEG and organic products having a boiling point of ≥ 224.8°C (1.013 bar) being discharged at the bottom below the part 5, organic products having a boiling point of > 255°C (1.013 bar) being discharged at the bottom below the parts 3 and 4, organic products having a boiling point of ≤ 224.8°C (1.013 bar) being discharged at the top and ADG being discharged from the middle part of the upper combined column region (1) and (2) (side offtake).

29. The process according to the preceding claim, wherein products separated off at the bottom below the part 5 are recirculated to the reaction of DEG with ammonia.

30. The process according to any of the five preceding claims, wherein the dividing wall column K60 has from 30 to 100 theoretical plates and is operated at a pressure in the range from 0.005 to 1 bar.

31. The process according to any of the six preceding claims, wherein the subregion of the column (DWC) divided by the dividing wall (DW) and comprising the subregions 3, 4 and 5 or 2, 3, 4 and 5 or in each case parts thereof is provided with ordered packing, random packing elements and/or trays and the dividing wall in these subregions is thermally insulating.

32. The process according to any of the seven preceding claims, wherein organic products separated off at the top are recirculated to the reaction of DEG with ammonia.

33. The process according to any of claims 16 to 32, wherein the ADG-comprising stream/streams separated off at the top of the columns K60 and/or K70 is/are fed in its/their entirety or in part to a column K80 in which ADG and organic products having a boiling point of ≥ 224.8°C (1.013 bar) are separated off at the bottom and organic products having a boiling point of ≤ 224.8°C (1.013 bar) are separated off at the top.

34. The process according to the preceding claim, wherein ADG is additionally separated off at a side offtake of the column K80.

35. The process according to either of the two preceding claims, wherein products separated off at the bottom of column K80 are recirculated to the feed to the columns K60 and/or K70.

36. The process according to any of the three preceding claims, wherein products separated off at the top of column K80 are discharged or recirculated to the reaction of DEG with ammonia.

37. The process according to any of the four preceding claims, wherein the column K80 has from 10 to 80 theoretical plates and is operated at a pressure in the range from 0.005 to 3 bar.

38. The process according to any of the five preceding claims, wherein the feed point of column K80 is located in the upper or middle third, based on the number of theoretical plates, and the ADG side offtake located opposite the feed point is located from 1 to 30 theoretical plates below the feed point.

39. The process according to any of the preceding claims for the fractionation of mixtures comprising morpholine (MO), monoaminodiglycol (ADG), ammonia, water, N-ethylmorpholine (E-MO), 1,2-ethylenediamine (EDA) and organic products having a boiling point of > 224.8°C (1.013 bar).

40. The process according to any of the preceding claims, wherein the mixture comprising morpholine (MO), monoaminodiglycol (ADG), ammonia and water has been obtained by reaction of diethylene glycol (DEG) with ammonia in the presence of a heterogeneous catalyst comprising Cu, Ni and Co on zirconium dioxide as support or in the presence of a heterogeneous catalyst comprising Cu and Ni on aluminum oxide as support.

41. The process according to any of the preceding claims for preparing morpholine (MO) having a purity of ≥ 99.5% by weight, an N-ethylmorpholine (E-MO) content of ≤ 0.20% by weight, a 1,2-ethylenediamine (EDA) content of ≤ 0.30% by weight, a 2-methoxyethanol content of < 0.50% by weight and a water content of ≤ 0.05% by weight.

42. The process according to any of the preceding claims for preparing morpholine (MO) having an APHA color number of ≤ 10 and a chloride content of
≤ 15 mg/liter.

43. The process according to any of claims 20 to 42 for preparing monoaminodiglycol (ADG) having a purity of ≥ 98.00% by weight, a DEG content of ≤ 0.40% by weight, a water content of ≤ 0.20% by weight and an APHA color number of ≤ 20.

44. The process according to any of claims 15 to 43 for preparing N-ethylmorpholine (E-MO) having a purity of ≤ 98.50% by weight, a water content of ≤ 0.30% by weight and an APHA color number of ≤ 50.

## Revendications

1. Procédé de séparation continue par distillation de mélanges contenant de la morpholine (MO), du monoaminodiglycol (ADG), de l'ammoniac et de l'eau, obtenus par réaction de diéthylèneglycol (DEG) avec de l'ammoniac, **caractérisé en ce que**
dans une première colonne de distillation K10, l'ammoniac est séparé par la tête, la sortie de fond de K10 est introduite dans une deuxième colonne de distillation K20, dans laquelle l'eau et les produits organiques sont séparés par la tête à une température de tête dans la plage allant de 45 à 198°C et à une pression dans la plage allant de 0,1 à 15 bars,
la sortie de fond de K20 est introduite dans une troisième colonne de distillation K30, dans laquelle la MO et les produits organiques ayant un point d'ébullition < 140 °C (1,013 bars) sont séparés par la tête ou par une sortie latérale et l'ADG et les produits organiques ayant un point d'ébullition > 190°C (1,013 bars) sont séparés par le fond, et
le courant contenant la MO, qui est séparé dans la colonne K30 par la tête ou par la sortie latérale, est introduit dans une colonne K40, dans laquelle la MO est séparée par une sortie latérale, les produits organiques ayant un point d'ébullition ≤ 128°C (1,013 bars) sont séparés par la tête et les produits organiques ayant un point d'ébullition ≥ 128 °C (1,013 bars) sont séparés par le fond.

2. Procédé selon la revendication précédente, **caractérisé en ce que** les produits séparés par le fond de la colonne K40 sont recyclés en totalité ou en partie dans l'alimentation de la colonne K30.

3. Procédé selon l'une quelconque des deux revendications précédentes, **caractérisé en ce que** les produits séparés par la tête de la colonne K40 sont recyclés en totalité ou en partie dans l'alimentation de la colonne K20.

4. Procédé selon l'une quelconque des revendications précédentes, **caractérisé en ce que** la colonne K10 comporte un nombre d'étapes de séparation théoriques dans la plage allant de 3 à 30, et est exploitée à une pression dans la plage allant de 5 à 30 bars.

5. Procédé selon l'une quelconque des revendications précédentes, **caractérisé en ce que** la colonne K20 comporte un nombre d'étapes de séparation théoriques dans la plage allant de 25 à 70, et est exploitée à une pression dans la plage allant de 0,1 à 10 bars.

6. Procédé selon l'une quelconque des revendications précédentes, **caractérisé en ce que** la colonne K30 comporte un nombre d'étapes de séparation théoriques dans la plage allant de 5 à 25, et est exploitée à une pression dans la plage allant de 0,01 à 5 bars.

7. Procédé selon l'une quelconque des revendications précédentes, **caractérisé en ce que** la colonne K40 comporte un nombre d'étapes de séparation théoriques dans la plage allant de 10 à 80, et est exploitée à une pression dans la plage allant de 0,01 à 12 bars.

8. Procédé selon l'une quelconque des revendications précédentes, **caractérisé en ce que** l'emplacement d'alimentation de la colonne K10 se trouve dans le tiers supérieur, par rapport au nombre d'étapes de séparation théoriques.

9. Procédé selon l'une quelconque des revendications précédentes, **caractérisé en ce que** l'emplacement d'alimentation de la colonne K20 se trouve dans le tiers intermédiaire, par rapport au nombre d'étapes de séparation théoriques.

10. Procédé selon l'une quelconque des revendications précédentes, **caractérisé en ce que** l'emplacement d'alimentation de la colonne K30 se trouve dans le tiers supérieur, par rapport au nombre d'étapes de séparation théoriques.

11. Procédé selon l'une quelconque des revendications précédentes, **caractérisé en ce que** l'emplacement d'alimentation de la colonne K40 se trouve dans le tiers supérieur ou intermédiaire, par rapport au nombre d'étapes de séparation théoriques, et la sortie latérale de MO opposée se trouve 1 à 30 étapes de séparation théoriques en dessous de l'emplacement d'alimentation.

12. Procédé selon l'une quelconque des revendications précédentes, **caractérisé en ce que** le courant contenant des produits aqueux et organiques, qui est séparé par la tête de la colonne K20, est introduit dans une colonne K50, dans laquelle une solution aqueuse de N-éthyl-morpholine (solution aqueuse de E-MO) est séparée par la tête ou par une sortie latérale liquide, et de l'eau est séparée par le fond.

13. Procédé selon la revendication précédente, **caractérisé en ce que** la colonne K50 comporte un nombre d'étapes de séparation théoriques dans la plage allant de 10 à 50, et est exploitée à une pression dans la plage allant de 0,1 à 16 bars.

14. Procédé selon l'une quelconque des deux revendications précédentes, **caractérisé en ce que** l'emplacement d'alimentation de la colonne K50 se trouve dans le tiers supérieur ou intermédiaire, par rapport au nombre d'étapes de séparation théoriques.

15. Procédé selon l'une quelconque des trois revendications précédentes, **caractérisé en ce que** la solution aqueuse de N-éthyl-morpholine est déshydratée, puis la phase organique ainsi formée est concentrée par distillation pour obtenir le produit de valeur.

16. Procédé selon l'une quelconque des revendications précédentes, **caractérisé en ce que** la sortie de fond de K30 est introduite dans une colonne de distillation K60, dans laquelle l'ADG est séparé par une sortie latérale, les produits organiques ayant un point d'ébullition ≤ 224,8 °C (1,013 bars) sont séparés par la tête et les produits organiques ayant un point d'ébullition > 255 °C (1,013 bars) sont séparés par le fond.

17. Procédé selon la revendication précédente, **caractérisé en ce que** la colonne K60 comporte un nombre d'étapes de séparation théoriques dans la plage allant de 20 à 80, et est exploitée à une pression dans la plage allant de 0,005 à 1 bar.

18. Procédé selon l'une quelconque des deux revendications précédentes, **caractérisé en ce que** l'emplacement d'alimentation de la colonne K60 se trouve dans le tiers intermédiaire ou inférieur, par rapport au nombre d'étapes de séparation théoriques, et la sortie latérale d'ADG opposée se trouve 1 à 30 étapes de séparation théoriques au-dessus de l'emplacement d'alimentation.

19. Procédé selon l'une quelconque des trois revendications précédentes, **caractérisé en ce que** les produits organiques séparés par la tête de la colonne K60 sont déchargés ou recyclés dans la réaction du DEG avec l'ammoniac.

20. Procédé selon l'une quelconque des quatre revendications précédentes, **caractérisé en ce que** le courant contenant l'ADG, qui est séparé par la sortie latérale de la colonne K60, est introduit dans une colonne K70, dans laquelle l'ADG est séparé par une sortie latérale, les produits organiques ayant un point d'ébullition ≥ 224,8 °C (1,013 bars) sont séparés par le fond et les produits organiques ayant un point d'ébullition ≤ 224,8 °C (1,013 bars) sont séparés par la tête.

21. Procédé selon la revendication précédente, **caractérisé en ce que** la colonne K70 comporte un nombre d'étapes de séparation théoriques dans la plage allant de 10 à 80, et est exploitée à une pression dans la plage allant de 0,005 à 1 bar.

22. Procédé selon l'une quelconque des deux revendications précédentes, **caractérisé en ce que** l'emplacement d'alimentation de la colonne K70 se trouve dans le tiers supérieur ou intermédiaire, par rapport au nombre d'étapes de séparation théoriques, et la sortie latérale d'ADG opposée se trouve 1 à 30 étapes de séparation théoriques au-dessus de l'emplacement d'alimentation.

23. Procédé selon l'une quelconque des trois revendications précédentes, **caractérisé en ce que** les produits séparés par le fond de la colonne K70 sont recyclés dans la réaction du DEG avec l'ammoniac.

24. Procédé selon l'une quelconque des quatre revendications précédentes, **caractérisé en ce que** les produits séparés par la tête de la colonne K70 sont recyclés dans la réaction du DEG avec l'ammoniac.

25. Procédé selon la revendication 16, **caractérisé en ce que** la colonne K60 est une colonne à paroi de séparation (TK).

26. Procédé selon la revendication précédente, **caractérisé en ce que** la colonne à paroi de séparation (TK) comporte une paroi de séparation (T) dans la direction longitudinale de la colonne, qui forme une zone de colonne commune supérieure (1), une zone de colonne commune inférieure (6), une zone d'alimentation (2, 4) comportant une zone d'enrichissement (2) et une zone de rectification (4), ainsi qu'une zone de soutirage (3, 5) comportant une zone d'enrichissement (3) et une zone de rectification (5), l'introduction de la sortie de fond de K30 ayant lieu dans le tiers supérieur ou intermédiaire de la zone d'alimentation (2, 4), par rapport au nombre d'étapes de séparation théoriques de la zone d'alimentation, le déchargement des produits organiques ayant un point d'ébullition > 255 °C (1,013 bars) ayant lieu par le fond, le déchargement des produits organiques ayant un point d'ébullition ≤ 224,8 °C (1,013 bars) ayant lieu par la tête, le déchargement de l'ADG ayant lieu à partir de la zone de colonne 1 et le déchargement des produits organiques gazeux ayant un point d'ébullition ≥ 224,8 °C (1,013 bars), tel que le DEG, ayant éventuellement lieu à partir du tiers supérieur ou intermédiaire de la zone de soutirage (3, 5) (sortie latérale), par rapport au nombre d'étapes de séparation théoriques de la zone de soutirage.

27. Procédé selon la revendication précédente, **caractérisé en ce que** les produits séparés par la sortie latérale gazeuse sont recyclés dans la réaction du DEG avec l'ammoniac.

28. Procédé selon la revendication 25, **caractérisé en ce que** la colonne à paroi de séparation (TK) comporte une paroi de séparation (T) dans la direction longitudinale de la colonne, qui forme une zone de colonne commune supérieure (1) et (2), une zone d'alimentation (3, 4) comportant une zone d'enrichissement (3) et une zone de rectification (4), ainsi qu'une zone (5), la paroi de séparation T étant formée jusqu'au fond de la colonne, l'introduction de la sortie de fond de K30 ayant lieu dans le tiers supérieur ou intermédiaire de la zone d'alimentation (3, 4), par rapport au nombre d'étapes de séparation théoriques de la zone d'alimentation, le déchargement du DEG et des produits organiques ayant un point d'ébullition ≥ 224,8 °C (1,013 bars) ayant lieu par le fond en dessous de la zone 5, le déchargement des produits organiques ayant un point d'ébullition > 255°C (1,013 bars) ayant lieu par le fond en dessous des zones 3 et 4, le déchargement des produits organiques ayant un point d'ébullition ≤ 224,8 °C (1,013 bars) ayant lieu par la tête et le déchargement de l'ADG ayant lieu à partir de la zone intermédiaire de la zone de colonne commune supérieure (1) et (2) (sortie latérale).

29. Procédé selon la revendication précédente, **caractérisé en ce que** les produits séparés par le fond en dessous de la zone 5 sont recyclés dans la réaction du DEG avec l'ammoniac.

30. Procédé selon l'une quelconque des cinq revendications précédentes, **caractérisé en ce que** la colonne à paroi de séparation K60 comporte un nombre d'étapes de séparation théoriques dans la plage allant de 30 à 100, et est exploitée à une pression dans la plage allant de 0,005 à 1 bar.

31. Procédé selon l'une quelconque des six revendications précédentes, **caractérisé en ce que** la partie de la colonne (TK) divisée par la paroi de séparation (T), constituée des parties 3, 4 et 5 ou 2, 3, 4 et 5, ou de chacune de leurs zones, est chargée avec des garnissages structurés, des corps de remplissage et/ou des plateaux, et la paroi de séparation de ces parties est conçue sous une forme calorifuge.

32. Procédé selon l'une quelconque des sept revendications précédentes, **caractérisé en ce que** les produits organiques séparés par la tête sont recyclés dans la réaction du DEG avec l'ammoniac.

33. Procédé selon l'une quelconque des revendications 16 à 32, **caractérisé en ce que** le ou les courants contenant l'ADG, qui sont séparés par la tête des colonnes K60 et/ou K70, sont introduits en totalité ou en partie dans une colonne K80, dans laquelle l'ADG et les produits organiques ayant un point d'ébullition ≥ 224,8 °C (1,013 bars) sont séparés par le fond et les produits organiques ayant un point d'ébullition ≤ 224,8 °C (1,013 bars) sont séparés par la tête.

34. Procédé selon la revendication précédente, **caractérisé en ce que** de l'ADG est également séparé par une sortie latérale de la colonne K80.

35. Procédé selon l'une quelconque des deux revendications précédentes, **caractérisé en ce que** les produits séparés par le fond de la colonne K80 sont recyclés dans l'alimentation des colonnes K60 et/ou K70.

36. Procédé selon l'une quelconque des trois revendications précédentes, **caractérisé en ce que** les produits séparés par la tête de la colonne K80 sont déchargés ou recyclés dans la réaction du DEG avec l'ammoniac.

37. Procédé selon l'une quelconque des quatre revendications précédentes, **caractérisé en ce que** la colonne K80 comporte un nombre d'étapes de séparation théoriques dans la plage allant de 10 à 80, et est exploitée à une pression dans la plage allant de 0,005 à 3 bars.

38. Procédé selon l'une quelconque des cinq revendications précédentes, **caractérisé en ce que** l'emplacement d'alimentation de la colonne K80 se trouve dans le tiers supérieur ou intermédiaire, par rapport au nombre d'étapes de séparation théoriques, et la sortie latérale de l'ADG opposée se trouve 1 à 30 étapes de séparation théoriques en dessous de l'emplacement d'alimentation.

39. Procédé selon l'une quelconque des revendications précédentes, pour la séparation de mélanges contenant de la morpholine (MO), du monoaminodiglycol (ADG), de l'ammoniac, de l'eau, de la N-éthyl-morpholine (E-MO), de la 1,2-éthylènediamine (EDA) et des produits organiques ayant un point d'ébullition > 224,8 °C (1,013 bars).

40. Procédé selon l'une quelconque des revendications précédentes, **caractérisé en ce que** le mélange contenant de la morpholine (MO), du monoaminodiglycol (ADG), de l'ammoniac et de l'eau est obtenu par réaction de diéthylèneglycol (DEG) avec de l'ammoniac en présence d'un catalyseur hétérogène contenant Cu, Ni et Co sur du dioxyde de zirconium en tant que support ou en présence d'un catalyseur hétérogène contenant Cu et Ni sur de l'oxyde d'aluminium en tant que support.

41. Procédé selon l'une quelconque des revendications précédentes, pour la fabrication de morpholine (MO) ayant une pureté ≥ 99,5 % en poids, une teneur en N-éthyl-morpholine (E-MO) s 0,20 % en poids, une teneur en 1,2-éthylènediamine (EDA) ≤ 0,30 % en poids, une teneur en 2-méthoxy-éthanol < 0,50 % en poids et une teneur en eau ≤ 0,05 % en poids.

42. Procédé selon l'une quelconque des revendications précédentes, pour la fabrication de morpholine (MO) ayant un indice de couleur APHA ≤ 10 et une teneur en chlorure ≤ 15 mg/litre.

43. Procédé selon l'une quelconque des revendications 20 à 42, pour la fabrication de monoaminodiglycol (ADG) ayant une pureté ≥ 98,00 % en poids, une teneur en DEG ≤ 0,40 % en poids, une teneur en eau ≤ 0,20 % en poids et un indice de couleur APHA ≤ 20.

44. Procédé selon l'une quelconque des revendications 15 à 43, pour la fabrication de N-éthyl-morpholine (E-MO) ayant une pureté ≥ 98,50 % en poids, une teneur en eau ≤ 0,30 % en poids et un indice de couleur APHA ≤ 50.
